# EUROPEAN PATENT SPECIFICATION

(11) **EP 2 094 675 B1**
(45) Date of publication and mention of the grant of the patent: **06.04.2011**
(21) Application number: 07825550.2
(22) Date of filing: 31.10.2007
(51) Int. Cl.: C07D 261/20, A61K 31/423, A61P 25/00

(54) **A SALT OF 3-BENZYL-2-METHYL-2,3,3A,4,5,6,7,7A-OCTAHYDROBENZO[D]ISOXAZOL-4-ONE**
SALZ VON 3-BENZYL-2-METHYL-2,3,3A,4,5,6,7,7A-OCTAHYDROBENZO[D]ISOXAZOL-4-ON
SEL DE 3-BENZYL-2-MÉTHYL-2,3,3A,4,5,6,7,7A-OCTAHYDROBENZO[D]ISOXAZOL-4-ONE

(30) Priority: 02.11.2006 IT MI20062102
(43) Date of publication of application: 02.09.2009
(73) Proprietor: Abiogen Pharma S.p.A., 56014 Località Ospedaletto (PI) (IT)
(72) Inventor: NEGGIANI, Fabio, 56123 Pisa (IT); DINI, Laura, 56127 Pisa (IT)
(74) Representative: Cattaneo, Elisabetta
(86) International application number: PCT/IB2007/003292
(87) International publication number: WO 2008/053326

(56) References cited:
- WO-A-93/17004

## Description

The present invention relates to oxalate salt of the compound of formula: 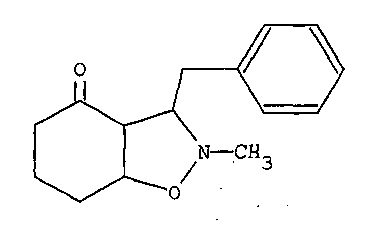 in all its stereochemical configurations, a process for its preparation and its use in treating mood disorders, disorders of anxiety, depression and convulsive conditions, in the improvement of learning ability, in the reversal of amnesia, in resolving the abstinence syndrome from drugs and drugs of abuse.

More specifically, the invention concerns oxalate salt of rel-(3R,3aS,7aS)-3-benzyl-2-methyl-2,3,3a,4,5,6,7,7a-octahydrobenzo[d]isoxazol-4-one.

The compound rel-(3R,3aS,7aS)-3-benzyl-2-methyl-2,3,3a,4,5,6,7,7a-octahydrobenzo[d]isoxazol-4-one, also known as BTG 1640, is described in the International application with publication number WO93/17004 and belongs to a family of new psychoactive compounds.

Specifically, in the patent application with publication number WO93/17004 the tranquilizing activity of the whole compound family is proposed and particularly, the activity of the compound BTG 1640 and the effects thereof in learning ability and in the reversal of amnesia are tested.

According to such a document, the compound BTG 1640 is prepared as a yellow oil and, in tests demonstrating the proposed activity, it is used after dilution in PEG and distilled water.

In pharmaceutical practises, it is known that oil substances have a lot of drawbacks mostly linked to the difficult handling and formulation. The oils are in fact hard to weight, basically less stable to temperature variations, less soluble in ordinary solvents and therefore technically harder to dose for the preparation of pharmaceutical formulations.

It is generally advisable that the form of active ingredient is solid one which normally shows better characteristics, specifically in terms of handling for activities of pharmaceutical formulation.

With regard to this, the patent application WO93/17004 cites the possibility of preparing the salt form of new psychoactive compounds of general Formula (I), by treating the free base of a compound of Formula (I) with the suitable free acid. Specifically, document WO93/17004 describes the hydrochloride of BTG 1640 obtained in the form of white crystalline powders.

By taking such a salt as a reference, and wishing therefore to avoid the use of free base BTG 1640 in oil form, in the step of drug formulation, the stability of such a hydrochloride salt as active principle was evaluated, in order to state the storage and preservation conditions of the drug. It is known that a pharmaceutical substance is deemed stable if it maintains the same initial characteristics, when subjected to different temperature and humidity conditions in time. As it will be demonstrated in the examples, the stability analysis showed that the hydrochloride salt of BTG 1640 is unstable at temperatures higher than 30°C, thereby refrigerator preservation at temperatures of 2-8°C becomes advisable, in precautional way, not only for the single salt as active substance, but also for the different pharmaceutical formulations, which are nowadays developed and based on such an active principle.

Such a refrigerator preservation condition, although necessary, results rather disadvantageous. As a matter of fact, the observation of such preservation precautions is scarcely accepted by the patient, who results evidently not accommodated in the daily practise and in his/her activities, by having to keep the drug in the refrigerator, out of necessity.

It is still felt the need of providing the compound 3-benzyl-2-methyl-2,3,3a,4,5,6,7,7a-octahydrobenzo[d]isoxazol-4-one in a form which does not require peculiar preservation conditions.

An object of the present invention is therefore to provide a form of 3-benzyl-2-methyl-2,3,3a,4,5,6,7,7a-octahydrobenzo[d]isoxazol-4-one which does not require peculiar preservation and storage conditions.

It is a further object to provide a salt of BTG 1640 which shows bioavailability characteristics at least comparable to those of hydrochloride salt of known BTG 1640.

The inventors of the present invention carried out studies and researches and as a result they surprisingly found out that the oxalate salt of: 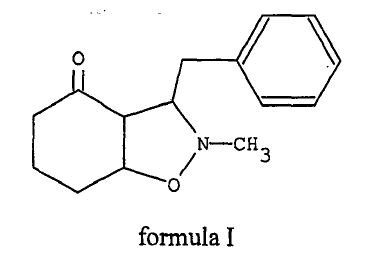 shows a highly improved stability so as not to require peculiar preservation conditions, even after six months of preservation at-40°C/75%RH.

Therefore the invention concerns oxalate salt as recited in claim 1, a new process of preparation and its use as a medicament, specifically in treating mood disorders, disorders of anxiety, depression and convulsive conditions, in the improvement of learning ability, in the reversal of amnesia, in resolving the abstinence syndrome from drugs and drugs of abuse.

Preferably, according to the invention oxalate of rel-(3R,3aS,7aS)-3-benzyl-2-methyl-2,3,3a,4,5,6,7,7a-octahydrobenzo[d]isoxazol-4-one is provided, comprising the molecule in the two enantiomeric forms and as racemic mixture.

Advantages and further characteristics are indicated in the dependent claims.

In the present invention the molecule 3-benzyl-2-methyl-2,3,3a,4,5,6,7,7a-octahydrobenzo[d]isoxazol-4-one so as represented in formula I: 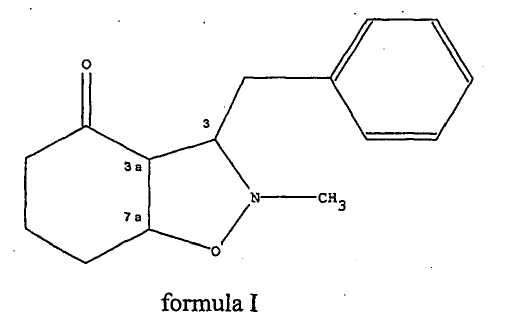 comprises in its definition any stereochemical configuration associated to chiral centers, as well as racemic mixtures and enantiomers obtainable through separation techniques known to skilled men and any mixture of two or more stereochemical compounds.

The invention will be now described in greater detail, particularly by making reference to the annexed figures wherein:
figure 1 is a graph of the results obtained in the stability tests carried out on BTG 1640 hydrochloride in temperature and humidity conditions of 5°C, 25°C/60% RH, 30°C/65%RH and 40°C/75%RH with reference to the purity of the examined substance;
figure 2 is a graph of the results obtained in the stability tests carried out on BTG 1640 hydrochloride in temperature and humidity conditions of 5°C, 25°C/60% RH, 30°C/65%RH and 40°C/75%RH with reference to the evaluation of total impurities;
figure 3 is a graph of the results obtained in the stability tests carried out on BTG 1640 oxalate in temperature and humidity conditions of 5°C, 25°C/60% RH, 30°C/65%RH and 40°C/75%RH with reference to the purity of the examined substance;
figure 4 is a graph of the results obtained in the stability tests carried out on BTG 1640 oxalate in temperature and humidity conditions of 5°C, 25°C/60% RH, 30°C/65%RH and 40°C/75%RH with reference to the evaluation of total impurities;
figure 5 is a graph of the results obtained in the stability tests carried out at temperatures of 5°C on BTG 1640 methanesulphonate, maleate, succinate, hydrochloride and oxalate with reference to the purity of the examined substance;
figure 6 is a graph of the results obtained in the stability tests carried out on BTG 1640 methanesulphonate, maleate, succinate, hydrochloride, and oxalate at temperature of 25°C and humidity of 60%RH with reference to the purity of the examined substance;
figure 7 is a graph of the results obtained in the stability tests carried out on BTG 1640 methanesulphonate, maleate, succinate, hydrochloride and oxalate at temperature of 30°C and humidity of 65%RH with reference to the purity of the examined substance;
figure 8 is a graph of the results obtained in the stability tests carried out on BTG 1640 methanesulphonate, maleate, succinate, hydrochloride and oxalate at temperature of 40°C and humidity of 75%RH with reference to the purity of the examined substance;
figure 9 is a graph of the results obtained in the stability tests carried out at temperature of 5°C on BTG 1640 methanesulphonate, maleate, succinate, hydrochloride and oxalate with reference to the evaluation of total impurities;
figure 10 is a graph of the results obtained in the stability tests carried out at temperature of 25°C and humidity of 60%RH on BTG 1640 methanesulphonate, maleate, succinate, hydrochloride and oxalate with reference to the evaluation of total impurities;
figure 11 is a graph of the results obtained in the stability tests carried out at temperature of 30°C and humidity of 65%RH on BTG 1640 methanesulphonate, maleate, succinate, hydrochloride and oxalate with reference to the evaluation of total impurities;
figure 12 is a graph of the results obtained in the stability tests carried out at temperature of 40°C and humidity of 75%RH on BTG 1640 methanesulphonate, maleate, succinate, hydrochloride and oxalate with reference to the evaluation of total impurities;
figure 13 is a graph of the results of the absorption kinetics in Sprague Dawley rats subjected to oral administration in dosages of 10 mg/kg (as expressed as free base of BTG 1640) of a formulation containing either hydrochloride salt or oxalate salt of BTG 1640 dispersed in an aqueous suspension of arabic gum at 5%.

The invention therefore concerns the oxalate salt of molecule 3-benzyl-2-methyl-2,3,3a,4,5,6,7,7a-octahydrobenzo[d]isoxazol-4-one.

According to the invention, such a salt can be obtained by treating the free base 3-benzyl-2-methyl-2,3,3a,4,5,6,7,7a-octahydrobenzo[d]isoxazol-4-one with oxalic acid, or alternatively, from hydrochloride salt of 3-benzyl-2-methyl-2,3,3a,4,5,6,7,7a-octahydrobenzo[d]isoxazol-4-one through treatment directed to free the molecule 3-benzyl-2-methyl-2,3,3a,4,5,6,7,7a-octahydrobenzo[d]isoxazol-4-one as free base and subsequent reaction with oxalic acid.

In another aspect the invention concerns a process for the preparation of oxalate salt of 3-benzyl-2-methyl-2,3,3a,4,5,6,7,7a-octahydrobenzo[d]isoxazol-4-one according to claim 5, comprising the following steps:
i) reacting the free base 3-benzyl-2-methyl-2,3,3a,4,5,6,7,7a-octahydrobenzo[d]isoxazol-4-one with oxalic acid;
ii) subjecting the reaction mixture to one or more cooling cycles below to 10°C.

Alternatively, the free base 3-benzyl-2-methyl-2,3,3a,4,5,6,7,7a-octahydrobenzo[d]isoxazol-4-one of step i) can be obtained in a preceding step of step i) which provides for freeing said base from the correspondent hydrochloride.

Specifically, the hydrochloride salt of 3-benzyl-2-methyl-2,3,3a,4,5,6,7,7a-octahydrobenzo[d]isoxazol-4-one can be subjected to subsequent extractions in dichloromethane in order to obtain the molecule 3-benzyl-2-methyl-2,3,3a,4,5,6,7,7a-octahydrobenzo[d]isoxazol-4-one as free base in form of a transparent oil of slightly brown colour. To such a solution oxalic acid can be added, preferably in dihydrate form and in presence of an ice bath in order to separate the oxalate salt, after a cooling cycle at about 2-8°C, in form of crystals. Further processes of preparation of salt can by provided for by a synthesis organic technician. The salt so obtained can furthermore be optionally subjected to purification methods, if necessary.

The oxalate salt so obtained resulted to be stable in different preservation conditions, as it will be demonstrated in examples, thus demonstrating itself more stable than hydrochloride salt.

The salt oxalate of 3-benzyl-2-methyl-2,3,3a,4,5,6,7,7a-octahydrobenzo[d]isoxazol-4-one resulted, surprisingly, not only more stable than the known hydrochloride, but, as it will be widely demonstrated in the following, it resulted more bioavailable, that is it showed a kinetic absorption profile *in vivo* better than the hydrochloride salt.

Furthermore, the oxalate salt of 3-benzyl-2-methyl-2,3,3a,4,5,6,7,7a-octahydrobenzo[d]isoxazol-4-one resulted surprisingly less toxic than hydrochloride salt as it will be shown below.

Oxalate of the compound of formula I having improved stability can be combined to suitable excipients for formulating pharmaceutical compositions according to the invention and is capable to act as pharmaceutical active substance in the treatment of mood disorders, disorders of anxiety, depression and convulsive conditions, in the improvement of learning ability, in the reversal of amnesia generated for example by Alzheimer disease or vascular dementia, in resolving the abstinence syndrome from drugs and drugs of abuse.

The daily dose required to reach the effect in the treatment of the indicated pathology varies with the subject, by depending by age, body weight and health general state, but it can be provided for a dosage suitable for the oral or topic administration in the range from 1 to 100 mg, once or more times a day, and a dosage suitable for parental administration in the range from 0.1 to 100 mg, once or more times a day.

The oxalate salt of compound of formula I will be added to a pharmaceutically acceptable carrier and, optionally, to other excipients in order to obtain pharmaceutical compositions to be parenterally, orally or topically administered. By the term "pharmaceutically acceptable carrier" it is meant to include solvents, supporting agents, diluents and the like, which are used as additives in order to provide a carrier suitable to the administration of the salt of the invention.

Compositions of the present invention suitable for oral administration will be conveniently in the form of discrete units such as tablets, capsules, cachets, powders, or granules, or still as suspensions in a liquid.

More preferably, the composition of the invention for the oral administration will be in form of tablets.

The tablet according to the invention comprises preferably an amount from 1 to 100 mg, preferably from 1 to 50 mg, of oxalate salt of the compound of formula I per tablet unit. Advantageously, the tablet comprises also suitable excipients, such as pre-gel starch, microcrystalline cellulose, sodium starch glycolate, talc, lactose, magnesium stearate, sucrose, stearic acid and mannitol.

Preferably, the tablet comprises from 1.7% to 40% by weight of oxalate of 3-benzyl-2-methyl-2,3,3a,4,5,6,7,7a-octahydrobenzo[d]isoxazol-4-one, more preferably from 2.1% to 34.7% by weight with respect of the total weight of the tablet.

The composition for oral administration preferably will comprise from 1 to 100 mg of oxalate salt of 3-benzyl-2-methyl-2,3,3a,4,5,6,7,7a-octahydrobenzo[d]isoxazol-4-one.

Compositions for the parenteral administration will comprise conveniently sterile aqueous preparations.

The compositions for parenteral administration preferably wil 0.1 to 100 mg of oxalate salt of 3-benzyl-2-methyl-2,3,3a,4,5,6,7,7a-octahydrobenzo[d]isoxazol-4-one.

Compositions for topical administration will be conveniently in form of creams, oils, ointments, emulsions, gels, aqueous solutions, spray solutions and plasters.

The compositions for topical administration preferably will comprise from 1 to 100 mg of oxalate of 3-benzyl-2-methyl-2,3,3a,4,5,6,7,7a-octahydrobenzo[d]isoxazol-4-one.

The invention will now described in greater details in the following examples, by way of non-limiting example of the invention and relating to the preparation of oxalate of BTG 1640 and to the evaluation of the stability, absorption efficacy and toxicity of the hydrochloride salt of the prior art and of the oxalate salt of the invention.

### Example 1. Preparation of oxalate of BTG 1640

### A. Preparation of the free base

10g of BTG 1640 hydrochloride were solubilized in 70 ml of sodium bicarbonate at 5%. The obtained solution was transferred into a separatory funnel of 250 ml and 50 ml of dichloromethane were added. After strong stirring for 30 seconds, the solution was left to rest until the separation of two layers. The phase containing dichloromethane was then transferred in an Erlenmeyer flask and further 20 ml of dichloromethane were added to the separatory funnel. Strong stirring of the solution contained in the separatory funnel was then again carried out for 30 seconds and the solution was left to rest until a new separation of the two layers. Once dichloromethane phase was separated, which was added to the Erlenmeyer flask, the extraction with further 20 ml of dichloromethane was repeated. Three aliquots of dichloromethane were then anhydrified with sodium solphate, filtered on paper filter and evaporated at rotavapor at a temperature below 35°C. The residue obtained as a slightly brown coloured oil corresponded to the free base of BTG 1640.

### B. Preparation of oxalate salt

1 g of free base BTG 1640 obtained as indicated in part A was then solubilized in 20 ml of methanol. 10 ml of a methanolic solution comprising 514 mg of dihydrate oxalic acid (PM= 126.07) were then added dropw addition, the solution of BTG 1640 was kept submerged in an bath ice.

5 ml of hexane (alternatively 5ml of ether can be employed) were then added and the solution was transferred into refrigerator at about 2-8°C for at least 48 hours. At the end of 48 hours, the formed crystals were isolated through filtration and finally dried into a stove under vacuum at 35°C. Mother liquor was then concentrated to a residue and during such an operation other crystals were formed. This solution was left 48 hours in the refrigerator at the temperature of 2-8°C and thereafter the formed crystals were isolated through filtration and finally dried into a stove under vacuum at 35°C. Mother liquor was again concentrated to a residue and such a residue was then retaken with 55 ml of diisopropilic ether and 30 ml of ethyl acetate. The obtained solution was again placed into the refrigerator at 2-8°C for 48 hours. At the end of 48 hours the formed crystals were isolated through filtration and finally dried into a stove under vacuum at 35°C. The overall yield of the process was 68%.

The so obtained crystals were subjected to characterization in order to demonstrate they corresponded to oxalate of BTG 1640.

A sample of crystal was then subjected to elementary analysis and the obtained results corresponded to the values calculated on the basis of the proposed chemical formula.

### Oxalate of BTG 1640: C₁₆H₂₀NO₄

| **Element** | **Theoric** | **Found** |
|---|---|---|
| C% | 66.19 | 66.37 |
| H% | 6.94 | 7.15 |
| N% | 4.82 | 4.41 |
| O% | 22.05 | 22.07 |

The spectrum 1H-NMR, which confirmed the formation of oxalate of BTG 1640, was then obtained.

¹H-NMR (200 MHz, DMSO-d6) δ: 1.63-2.60 (m, 6H) for H5 H6 e H7; 2.41 (s, 3H) for N-CH3; 2.75-2.82 (m, 2H) for benzylic CH2; 2.85-2.95 (m, 1H) for H3a; 3.38 (br, 1H) H3; 4.30 (br, 1H) for H7a; 7.15-7.40 (m, 5H) for aromatic structure.

### Example 2

### Analysis of the stability of hydrochloride of BTG 1640

Samples of 100 mg each of BTG 1640 hydrochloride were subjected to the following preservation conditions:
a) 51C;
b) 25°C and 60%RH;
c) 30°C and 65%RH;
d) 40°C and 70%RH.
The purities were then analyzed periodically, initially every three months, in order to underline possible variations.

The results in the following Table 1 were obtained:

**Table 1: Purity % of BTG 1640 hydrochloride**

| Time | T=5°C | T=25°C Humidity=60%RH | T=30°C Humidity=65%RH | T=40°C Humidity=75%RH |
|---|---|---|---|---|
| 0 | 99.3 | 99.7 | 99.7 | 99.7 |
| 3 | 100.1 | 100.9 | 100.0 | 93.4 |
| 6 | 100.3 | 101.5 | 100.9 | 81.2 |
| 9 | 99.6 | 99.3 | 100.6 | |
| 12 | 99.6 | 101.1 | 99.9 | |
| 18 | 100.8 | 100.5 | | |
| 24 | 98.8 | 99.3 | | |
| 36 | 99.8 | 99.5 | | |

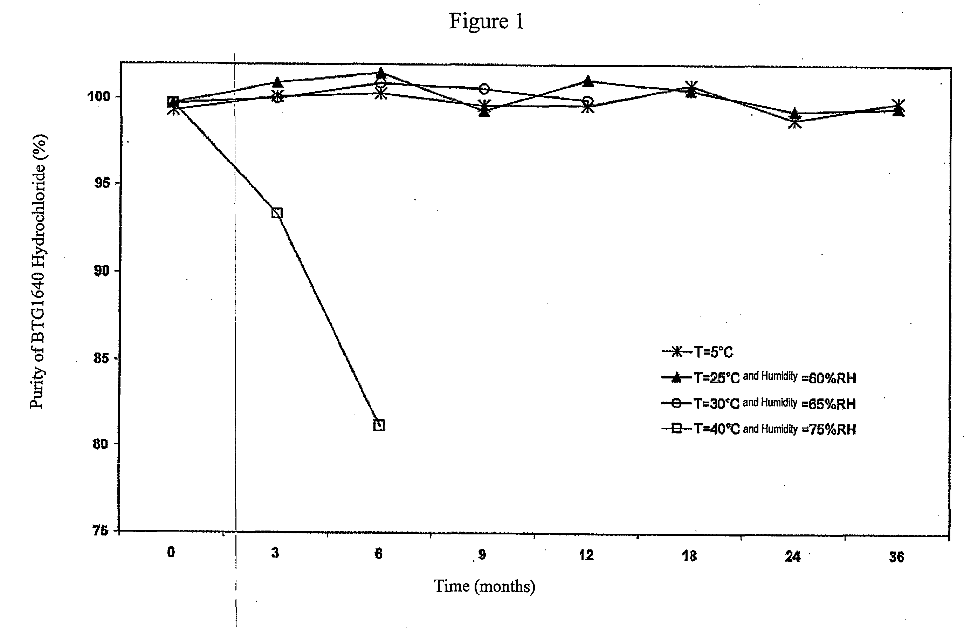

The obtained results have been represented in figure 1. As it is seen in figure 1, BTG 1640 hydrochloride showed a good stability for the T/RH conditions indicated at points a)-c) for a time period of 12 months, while it showed a strong instability since the first months, when subjected to 40°C and 75%RH, thus demonstrating that conditions d) determined a decomposition of the salt, already at six months. At the end of the test, on the basis of the results, the drug was indicated as having a validity time period of 12 months and, precautionally, preferably preserved at temperatures between 2 and 8°C, because of the strong degradation occurred in conditions 40°C/75%RH.

Contemporaneously, the impurity analysis in the conditions a)-d) of T/%RH were carried out.

The results in the following Table 2 were obtained:

**Table 2: Calculation of impurities of BTG 1640 hydrochloride:**

| Time | T=5°C | T=25°C e Humidity=60%RH | T=30°C e Humidity=65%RH | T=40°C e Humidity=75%RH |
|---|---|---|---|---|
| 0 | 0.36 | 0.36 | 0.36 | 0.36 |
| 3 | 0.58 | 0.43 | 0.44 | 6.99 |
| 6 | 0.45 | 0.87 | 0.52 | 17.31 |
| 9 | 0.31 | 0.24 | 0.12 | |
| 12 | 0.31 | 0.34 | 0.76 | |
| 18 | 0.1 | 0.13 | | |
| 24 | 0.21 | 0.28 | | |
| 36 | 0.18 | 0.30 | | |

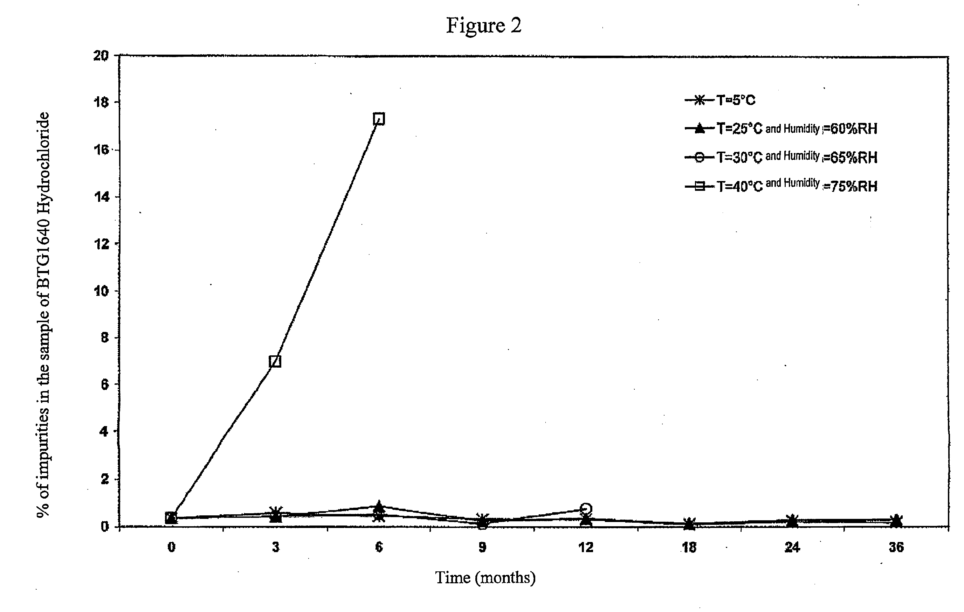

The results were then represented in figure 2, from which it was shown that BTG 1640 hydrochloride in condition d) showed, already after three months of stability at 40°C/75%RH, an unacceptable percentage of impurities.

### Example 3

### Analysis of stability of oxalate of BTG 1640

Samples of 100 mg each of BTG 1640 oxalate prepared according to example 1B were subjected to the following preservation conditions:
a) 5°C;
b) 25°C and 60%RH;
c) 30°C and 65%RH;
d) 40°C and 75%RH.
Quarterly and in an overall period of nine months its purity was then analyzed in order to point out possible variations.

The results in the following Table 3 were obtained:

**Table 3: Purity % of BTG 1640 oxalate**

| Time | T=5°C | T=25°C Humidity=60%RH | T=30°C Humidity=65%RH | T=40°C Humidity=75%RH |
|---|---|---|---|---|
| 0 | 99.7 | 99.7 | 99.7 | 99.7 |
| 3 | 99.7 | 99.7 | 99.7 | 100.0 |
| 6 | 100.0 | 100.0 | 100.0 | 100.0 |
| 9 | 99.7 | 99.9 | 99.9 | |

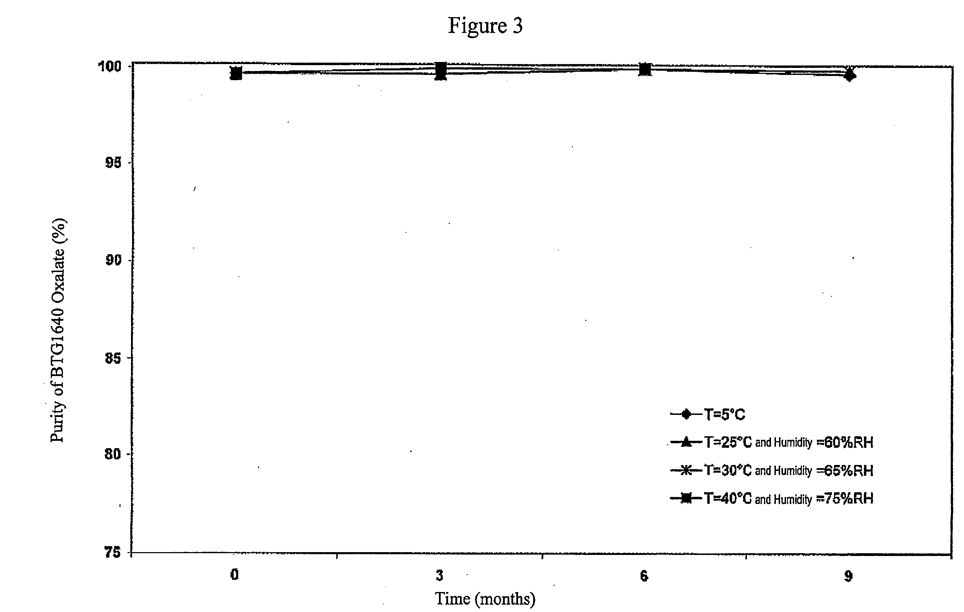

The results were represented in figure 3. As it is seen from Figure 3, BTG 1640 oxalate, similarly to BTG 1640 hydrochloride, showed a go T/RH conditions indicated at points a)-c) for the overall period, and such a stability, contrary to salt BTG 1640 hydrochloride, was maintained unaltered for the overall period-of observation, also in the severer condition of point d), thus demonstrating the higher stability of oxalate salt in compare to hydrochloride salt.

As a confirmation of such evidence, a test for the evaluation of the impurity percentage of the sample subjected to the same conditions a)-d) of T/%RH was parallely carried out. The experimental values in Table 4 were obtained.

**Table 4: Calculation of impurities of BTG 1640 oxalate**

| Time | T=5°C | T=25°C Humidity=60%RH | T=30°C Humidity=65%RH | T=40°C Humidity=75%RH |
|---|---|---|---|---|
| 0 | 0.27 | 0.27 | 0.27 | 0.27 |
| 3 | 0.33 | 0.30 | 0.28 | 0.10 |
| 6 | 0.10 | 0.10 | 0.10 | 0.10 |
| 9 | 0.29 | 0.10 | 0.10 | |

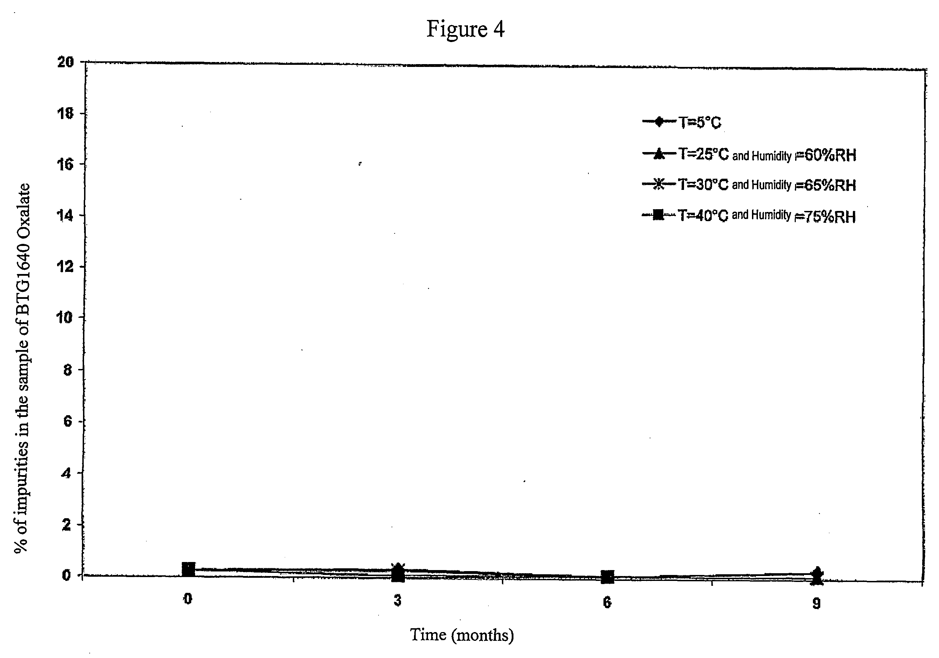

The obtained results were then represented in figure 4.

As it is seen from figure 4, BTG 1640 oxalate, also in conditions d) and until the sixth month of observation, kept to show an invariable impurity profile with respect to time zero, thus being optimal and therefore, contrary to hydrochloride quite acceptable.

Therefore it is evident from the tests carried out the higher stability of the oxalate salt in compare to hydrochloride salt of BTG 1640.

### Example 4

### Analysis of the stability of various salts of BTG 1640

As comparison other salts of BTG 1640 were prepared, and exactly:
- BTG 1640 methanesulphonate
- BTG 1640 maleate
- BTG 1640 succinate
following the procedure indicated in example 1 and substituting methanesolphonic, maleic and succinic acids for oxalic acid.

The stabilities were then evaluated by subjecting samples of 100 mg of each salt to the following analogous preservation conditions:
a) 5°C;
b) 25°C and 60%RH;
c) 30°C and 65%RH;
d) 40°C and 70%RH.

Quarterly and in an overall period of nine months their purity was then analyzed in order to underline possible variations.

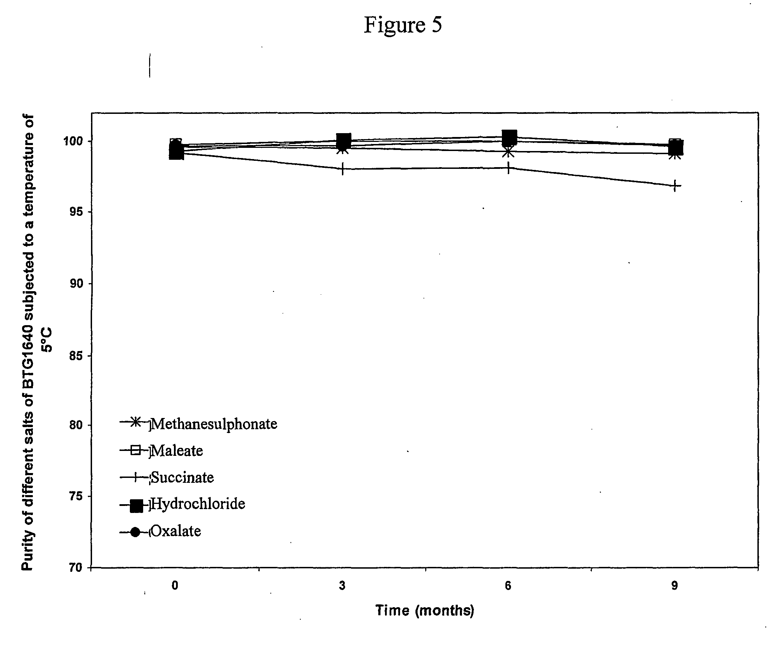

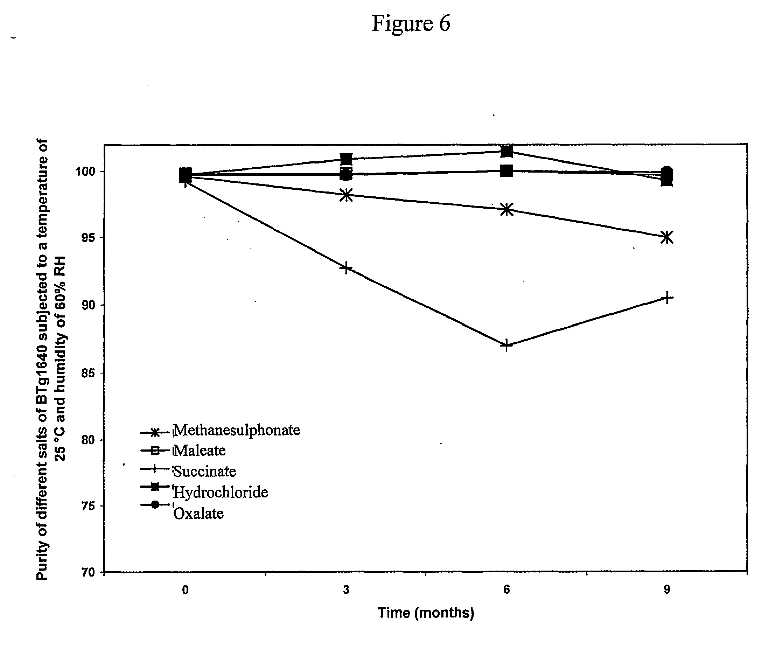

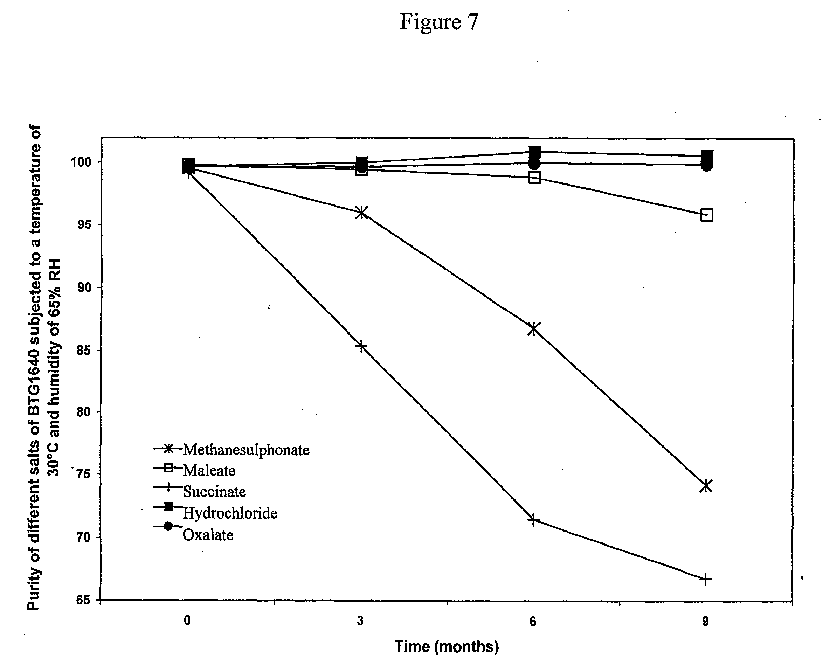

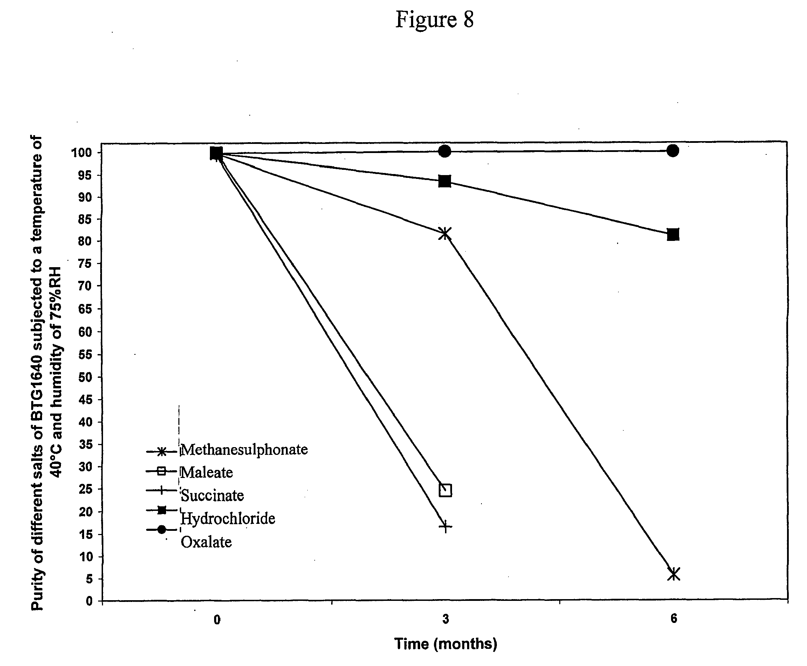

The obtained results, together with those obtained in Example 2 for hydrochloride and in Example 3 for oxalate in order to make easier the comparison, were in Table 5 and represented in figures 5-8.

**Table 5: Comparison of stabilities of different salts of BTG 1640**

| Purity data % of various salts of BTG 1640 at temperature of 5°C | | | | | |
|---|---|---|---|---|---|
| Time | Methanesulphonate | Maleate | Succinate | Hydrochloride | Oxalate |
| 0 | 99.6 | 99.8 | 99.2 | 99.3 | 99.7 |
| 3 | 99.5 | 100.0 | 98.0 | 100.1 | 99.7 |
| 6 | 99.3 | 100.0 | 98.1 | 100.3 | 100.0 |
| 9 | 99.1 | 99.8 | 96.8 | 99.6 | 99.7 |

| Purity data % of various salts of BTG 1640 at temperature of 25°C and relative humidity of 60% | | | | | |
|---|---|---|---|---|---|
| Time | Methanesulphonate | Maleate | Succinate | Hydrochloride | Oxalate |
| 0 | 99.6 | 99.8 | 99.2 | 99.7 | 99.7 |
| 3 | 98.2 | 99.8 | 92.7 | 100.9 | 99.7 |
| 6 | 97.1 | 100.0 | 87.0 | 101.5 | 100.0 |
| 9 | 95.0 | 99.7 | 90.5 | 99.3 | 99.9 |

| Purity data% of various salts of BTG 1640 at temperature of 30°C and relative humidity of 65% | | | | | |
|---|---|---|---|---|---|
| Time | Methanesulphonate | Maleate | Succinate | Hydrochloride | Oxalate |
| 0 | 99.6 | 99.8 | 99.2 | 99.7 | 99.7 |
| 3 | 96.0 | 99.5 | 85.4 | 100.0 | 99.7 |
| 6 | 86.8 | 98.9 | 71.5 | 100.9 | 100.0 |
| 9 | 74.2 | 95.9 | 66.8 | 100.6 | 99.9 |

| Purity data % of various salts of BTG 1640 at temperature of 40°C and relative humidity of 75% | | | | | |
|---|---|---|---|---|---|
| Time | Methanesulphonate | Maleate | Succinate | Hydrochloride) | Oxalate |
| 0 | 99.6 | 99.8 | 99.2 | 99.7 | 99.7 |
| 3 | 81.5 | 24.4 | 16.5 | 93.4 | 100.0 |
| 6 | 5.7 | | | 81.2 | 100.0 |

As it is seen from figures 5-8 the stability profiles of salts methanesulphonate, maleate and succinate of BTG 1640 seem to be even worse than the stability profiles of BTG 1640 hydrochloride.

The methanesulphonate salt begins to degrade already after three months of stability in the cited conditions b)-d), in amount of course higher as the conditions become severer, i.e. from a) to d). Maleate salt shows an acceptable profile in conditions a) and b), while it shows considerable degradation if subjected to conditions c) already at sixth month and degrades in unacceptable way in condition d). Succinate salt, at the end, shows degradation at all observation conditions a)-d), already from the third month and considerably as higher as severer are preservation conditions, i.e. from a) to d).

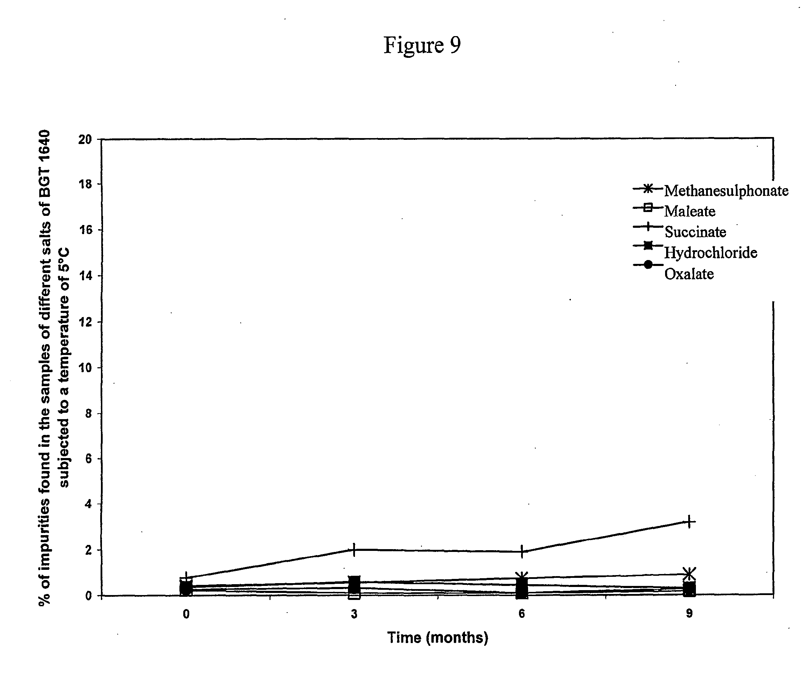

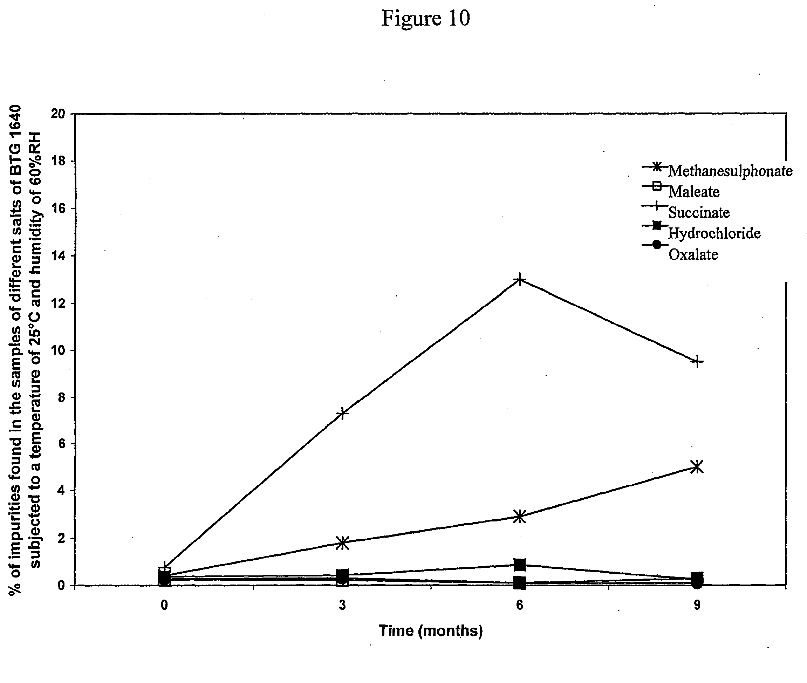

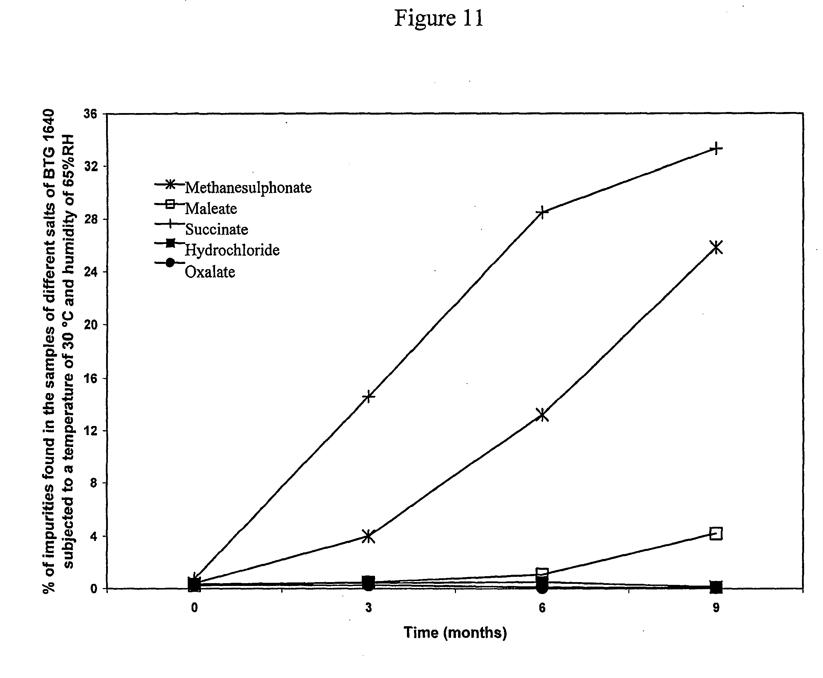

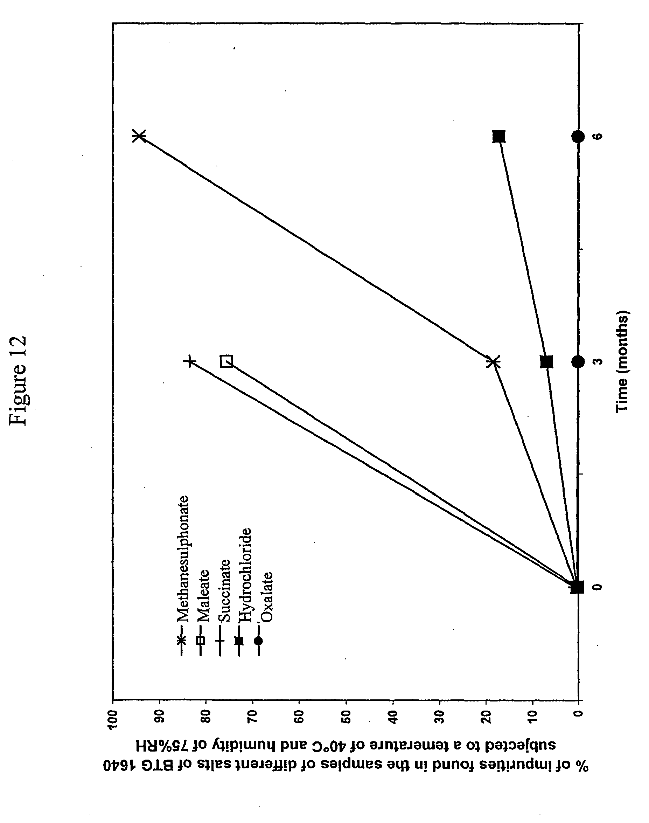

Consistently to these results, the impurity profile of salt samples subjected to such preservation conditions showed an unacceptable increase of the same as the salt itself degraded, as shown by patterns represented in figures 9-12, obtained on the basis of impurity results in Table 6.

**Table 6: Comparison of impurity amount of various salts of BTG 1640**

| **% impurities of various salts of BTG 1640 subjected to temperature of 5°C** | | | | | |
|---|---|---|---|---|---|
| Time | Methanesulphonate | Maleate | Succinate | Hydrochloride | Oxalate |
| 0 | 0.42 | 0.23 | 0.77 | 0.36 | 0.27 |
| 3 | 0.55 | 0.10 | 2.00 | 0.58 | 0.33 |
| 6 | 0.74 | 0.10 | 1.90 | 0.45 | 0.10 |
| 9 | 0.91 | 0.19 | 3.20 | 0.31 | 0.29 |

| **% impurities of various salts of BTG 1640 subjected t temperature of 25°C and relative humidity of 60%** | | | | | |
|---|---|---|---|---|---|
| Time | Methanesulphonate | Maleate | Succinate | Hydrochloride | Oxalate |
| 0 | 0.42 | 0.23 | 0.77 | 0.36 | 0.27 |
| 3 | 1.80 | 0.22 | 7.30 | 0.43 | 0.30 |
| 6 | 2.90 | 0.10 | 13.00 | 0.87 | 0.10 |
| 9 | 5.00 | 0.29 | 9.50 | 0.24 | 0.10 |

| **% impurities of various salts of BTG 1640 subjected to temperature of 30°C and relative humidity of 65%** | | | | | |
|---|---|---|---|---|---|
| Time | Methanesulphonate | Maleate | Succinate | Hydrochloride | Oxalate |
| 0 | 0.42 | 0.23 | 0.77 | 0.36 | 0.27 |
| 3 | 4.00 | 0.49 | 14.60 | 0.44 | 0.28 |
| 6 | 13.20 | 1.10 | 28.50 | 0.52 | 0.10 |
| 9 | 25.80 | 4.20 | 33.30 | 0.12 | 0.10 |

| **% impurities of various salts of BTG 1640 subjected to temperature of 40°C and relative humidity of 75%** | | | | | |
|---|---|---|---|---|---|
| Time | Methanesulphonate | Maleate | Succinate | Hydrochloride | Oxalate |
| 0 | 0.42 | 0.23 | 0.77 | 0.36 | 0.27 |
| 3 | 18.50 | 75.60 | 83.50 | 6.99 | 0.10 |
| 6 | 94.30 | | | 17.31 | 0.10 |

Surprisingly, therefore oxalate showed a different and improved stability with respect to known hydrochloride and other prepared acid addition salts, thus turning out to be the best ingredient for formulating pharmaceutical compositions, which does not require peculiar preservation conditions.

The present invention succeeded in solving the technical problem of obtaining a form of stable BTG 1640 through identification of the oxalate salt.

### Example 5.

### Analysis of absorption kinetic of oxalate and hydrochloride of BTG 1640

Absorption extent and rate of molecule BTG 1640 in its forms of oxalate and hydrochloride salts were evaluated.

With this intent, the two salts were dispersed in an aqueous suspension of arabic gum at 5% thus obtaining two formulations, each one orally administered through oesophageous gavage at dose of 10 mg/kg (expressed as free base of BTG 1640) to five rats Sprague Dawley.

Plasma concentration of molecule BTG 1640 was determined at different time points (see Table 7).

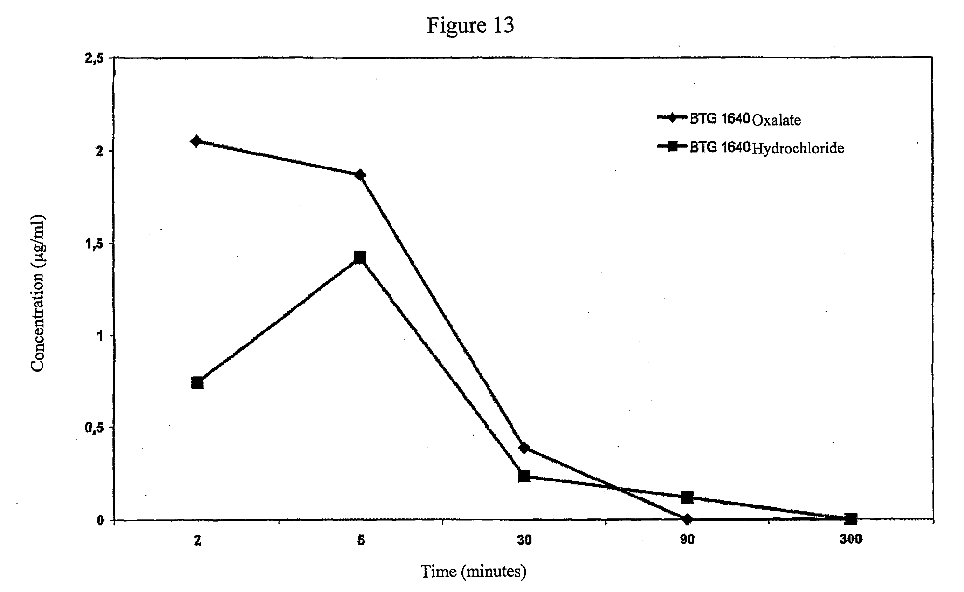

The results in Table 7 were obtained and represented in figure 13.

**Table 7: Concentration of oxalate and hydrochloride in Plasma.**

| *Time (minutes)* | BTG 1640 free base (µg/mL) with Oxalate Salt Administration | BTG 1640 free base (µg/mL) with Hydrochloride Salt Administration |
|---|---|---|
| 2 | 2.0523 | 0.7402 |
| 5 | 1.8673 | 1.4242 |
| 30 | 0.3897 | 0.2354 |
| 90 | 0 | 0.1184 |
| 300 | 0 | 0 |

From the pattern of the concentration profiles in time of molecule BTG 1640 in rat plasma, it is evident how oxalate salt provides an higher concentration of free and unmodified active principle BTG 1640 with respect to hydrochloride, after only two minutes from its administration.

This underlines therefore an higher absorption of oxalate salt in terms of both concentration and time.

Oxalate salt of BTG 1640 resulted therefore better than hydrochloride salt, not only in terms of profile of its chemical stability, but, for oral administration, also in terms of profile of its absorption kinetic in vivo.

### Example 6

### Evaluation of acute oral toxicity

Acute oral toxicity of salts hydrochloride and oxalate of BTG-1640 was evaluated.

The two salts were dispersed in an aqueous suspension, thus obtaining two formulations, each one orally administered through oesophageous gavage at dose of 2000 mg/kg bw (expressed as free base of BTG 1640) to one of two groups comprising three female CD-1 mice per group.

The observation lasted 14 days.

In all the animals of each group a reduction of the spontaneous locomotory activity was recorded in the first minutes after the treatment and quickly disappeared. Lethargy, ataxia, aggressiveness and vocalization were observed in the mice treated with hydrochloride BTG 1640 and two of them died in the first day following the administration of the entire dose. The body weight remained quite stable over the entire period of observation in all the experimental groups. Necropsy revealed the absence of alterations, anyway kidneys were sampled from each animal, in order to assess the possible renal toxicity of the salts. The histopathological examination of the group administered with hydrochloride showed small foci of acute tubular necrosis in the cortex, balloon degeneration of the epithelium and dilatation of convoluted tubules. In the group administered with oxalate a focal and moderate balloon degeneration of the epithelium in convoluted tubules was recorded. On the basis of the discussed results, the administration of 2000 mg/kg bw, in female mice, of oxalate BTG 1640 resulted less toxic, being followed by quickly reversible neurological signs, while the same dose of hydrochloride BTG 1640 was lethal in two animals out of three.

Some formulative examples for the preparation of tablets having weight of about 120 mg of pharmaceutical composition according to the invention comprising oxalate salt of BTG 1640 and directed to oral administration are indicated in the following.

### Example 7

| | |
|---|---|
| BTG 1640 oxalate | 2.6 mg |
| Sucrose | 81.4 mg |
| Microcrystalline cellulose | 20.4 mg |
| Talc | 9.6 mg |
| Stearic acid | 6.0 mg |

### Example 8

| | |
|---|---|
| BTG 1640 oxalate | 5.2 mg |
| Sucrose | 78.8mg |
| Microcrystalline cellulose | 20.4 mg |
| Talc | 9.6 mg |
| Stearic acid | 6.0 mg |

### Example 9

| | |
|---|---|
| BTG-1640 oxalate | 10.4 mg |
| Sucrose | 73.6 mg |
| Microcrystalline cellulose | 20.4 mg |
| Talc | 9.6 mg |
| Stearic acid | 6.0 mg |

### Example 10

| | |
|---|---|
| BTG 1640 oxalate | 20.8 mg |
| Sucrose | 63.2mg |
| Microcrystalline cellulose | 20.4 mg |
| Talc | 9.6 mg |
| Stearic acid | 6.0 mg |

### Example 11

| | |
|---|---|
| BTG 1640 oxalate | 41.6 mg |
| Sucrose | 42.4 mg |
| Microcrystalline cellulose | 20.4 mg |
| Talc | 9.6 mg |
| Stearic acid | 6.0 mg |

### Example 12

| | |
|---|---|
| BTG 1640 oxalate | 2.6 mg |
| Pre-gel starch | 24.0 mg |
| Microcrystalline cellulose | 76.6 mg |
| Sodium starch glycolate | 4.8 mg |
| Talc | 12.0 mg |

### Example 13

| | |
|---|---|
| BTG 1640 oxalate | 5.2 mg |
| Pre-gel starch | 24.0 g |
| Microcrystalline cellulose | 74.0 mg |
| Sodium starch glycolate | 4.8 mg |
| Talc | 12.0 g |

### Example 14

| | |
|---|---|
| BTG 1640 oxalate | 10.4 mg |
| Pre-gel starch | 24.0 mg |
| Microcrystalline cellulose | 69.2 mg |
| Sodium starch glycolate | 4.8 mg |
| Talc | 12.0 mg |

### Example 15

| | |
|---|---|
| BTG 1640 oxalate | 20.8 mg |
| Pre-gel starch | 24.0 mg |
| Microcrystalline cellulose | 58.4 mg |
| Sodium starch glycolate | 4.8 mg |
| Talc | 12.0 mg |

### Example 16

| | |
|---|---|
| BTG 1640 oxalate | 41.6 mg |
| Pre-gel starch | 24.0 mg |
| Microcrystalline cellulose | 37.6 mg |
| Sodium starch glycolate | 4.8 mg |
| Talc | 12.0 mg |

### Example 17

| | |
|---|---|
| BTG 1640 oxalate | 2.6 mg |
| Lactose | 73.8 mg |
| Microcrystalline cellulose | 43.0 mg |
| Magnesium stearate | 0.6 mg |

### Example 18

| | |
|---|---|
| BTG 1640 oxalate | 5.2 mg |
| Lactose | 73.8 mg |
| Microcrystalline cellulose | 40.4 mg |
| Magnesium stearate | 0.6 mg |

### Example 19

| | |
|---|---|
| BTG 1640 oxalate | 10.4 mg |
| Lactose | 73.8 mg |
| Microcrystalline cellulose | 35.2 mg |
| Magnesium stearate | 0.6 mg |

### Example 20

| | |
|---|---|
| BTG 1640 oxalate | 20.8 mg |
| Lactose | 73.8 mg |
| Microcrystalline cellulose | 24.8 mg |
| Magnesium Stearate | 0.6 mg |

### Example 21

| | |
|---|---|
| BTG 1640 oxalate | 41.6mg |
| Lactose | 73.8 mg |
| Microcrystalline cellulose | 4.0 mg |
| Magnesium Stearate | 0.6 mg |

### Example 22

| | |
|---|---|
| BTG 1640 oxalate | 2.6 mg |
| Mannitol | 24.0 mg |
| Microcrystalline cellulose | 76.6 mg |
| Sodium starch glycolate | 4.8 mg |
| Talc | 12.0 mg |

### Example 23

| | |
|---|---|
| BTG 1640 oxalate | 5.2 mg |
| Mannitol | 24.0 mg |
| Microcrystalline cellulose | 74.0 mg |
| Sodium starch glycolate | 4.8 mg |
| Talc | 12.0 mg |

### Example 24

| | |
|---|---|
| BTG 1640 oxalate | 10.4 mg |
| Mannitol | 24.0 mg |
| Microcrystalline cellulose | 69.2 mg |
| Sodium starch glycolate | 4.8 mg |
| Talc | 12.0 mg |

### Example 25

| | |
|---|---|
| BTG 1640 oxalate | 20.8 mg |
| Mannitol | 24.0 mg |
| Microcrystalline cellulose | 58.4 mg |
| Sodium starch glycolate | 4.8 mg |
| Talc | 12.0 mg |

### Example 26

| | |
|---|---|
| BTG 1640 oxalate | 41.6 mg |
| Mannitol | 24.0 mg |
| Microcrystalline cellulose | 37.6 mg |
| Sodium starch glycolate | 4.8 mg |
| Talc | 12.0 mg |

## Claims

1. Oxalate salt of the compound of formula I

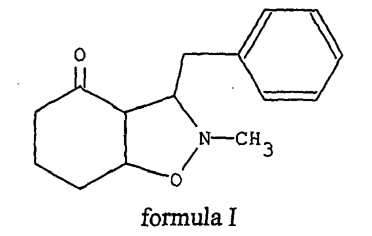

2. The oxalate salt of claim 1 which is oxalate salt of the compound of formula I in the form of one or more stereochemical compounds and mixtures thereof

3. The oxalate salt of claim 2 which is oxalate salt of the compound of formula I in the form of a diastereoisomer or a racemic mixture.

4. The oxalate salt according to any one of claims 1-3, which is oxalate of rel-(3R,3aS,7aS)-3-benzyl-2-methyl-2,3,3a,4,5,6,7,7a-octahydrobenzo[d]isoxazol-4-one.

5. A process for preparing oxalate salt of 3-benzyl-2-methyl-2,3,3a,4,5,6,7,7a-octahydrobenzo[d]isoxazol-4-one, according to any one of claims 1-4, comprising the following steps:
i) reacting the free base 3-benzyl-2-methyl-2,3,3a,4,5,6,7,7a-octahydrobenzo[d]isoxazol-4-one with oxalic acid;
ii) subjecting the reaction mixture to one or more cooling cycles of less than 10°C.

6. The process according to claim 5, wherein the free base 3-benzyl-2-methyl-2,3,3a,4,5,6,7,7a-octahydrobenzo[d]isoxazol-4-one of step i) is obtained in a step being previous to step i), which provides for freeing the said base from the corresponding hydrochloride salt.

7. The process according to claim 6, wherein freeing of the free base from the hydrochloride salt is carried out by dichloromethane.

8. The process according to any one of claims 5-7, wherein the oxalic acid of step i) is in dihydrate form.

9. The process according to any one of claims 5-8, wherein the step i) is carried out in an ice bath.

10. The process according to any one of claims 5-9, wherein the cooling of step ii) occurs at temperatures from 2 to 8°C.

11. Oxalate salt of the compound of formula I 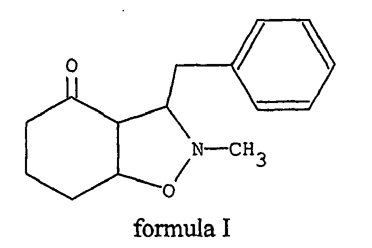 for use as a medicament.

12. The oxalate salt of claim 11 which is oxalate salt of the compound of formula I in the form of one or more stereochemical compounds and mixtures thereof for use as a medicament.

13. The oxalate salt of claim 12 which is oxalate salt of the compound of formula I in the form of a diastereoisomer or a racemic mixture for use as a medicament.

14. The oxalate salt according to any one of claims 11-13, which is oxalate of rel-(3R,3aS,7aS)-3-benzyl-2-methyl-2,3,3a,4,5,6,7,7a-octahydrobenzo[d]isoxazol-4-one for use as a medicament.

15. A pharmaceutical composition comprising the oxalate salt according to any one of claims 1-4 and a pharmaceutically acceptable carrier.

16. The composition according to claim 15, wherein the oxalate salt is present in an amount of from 0.1 to 100 mg.

17. A tablet comprising the oxalate salt according to any one of claims 1-4 and suitable excipients.

18. The tablet according to claim 17, wherein the suitable excipients are selected from the group consisting of pre-gel starch, microcrystalline cellulose, sodium starch glycolate, talc, lactose, magnesium stearate, saccarose, stearic acid and mannitol.

19. The tablet according to claim 17 or claim 18, wherein the oxalate salt is present in an amount of from 1 to 100 mg per tablet unit.

20. The tablet according to claim 19, wherein the oxalate salt is present in an amount of from 1 to 50 mg per tablet unit.

21. Use of the oxalate salt of the compound of formula I 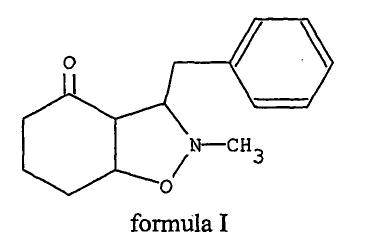 for the manufacture of a medicament for treating mood disorders, disorders of anxiety, depression and convulsive conditions, in the improvement of learning ability, in the reversal of amnesia, in resolving the abstinence syndrome from drugs and drugs of abuse.

22. The use according to claim 21, wherein the oxalate salt is oxalate of the compound of formula I in the form of one or more stereochemical compounds and mixtures thereof for the manufacture of a medicament for treating mood disorders, disorders of anxiety, depression and convulsive conditions, in the improvement of learning ability, in the reversal of amnesia, in resolving the abstinence syndrome from drugs and drugs of abuse.

23. The use according to claim 22, wherein the oxalate salt is oxalate of the compound of formula I in the form of a diastereoisomer or a racemic mixture for the manufacture of a medicament for treating mood disorders, disorders of anxiety, depression and convulsive conditions, in the improvement of learning ability, in the reversal of amnesia, in resolving the abstinence syndrome from drugs and drugs of abuse.

24. The use according to any one of claims 21-23, wherein the oxalate salt is oxalate of rel-(3R,3aS,7aS)-3-benzyl-2-methyl-2,3,3a,4,5,6,7,7a-octahydrobenzo[d]isoxazol-4-one for the manufacture of a medicament for treating mood disorders, disorders of anxiety, depression and convulsive conditions, in the improvement of learning ability, in the reversal of amnesia, in resolving the abstinence syndrome from drugs and drugs of abuse.

25. The use according to any one of claims 21-24, wherein the medicament is for oral administration.

26. The use according to claim 25, wherein the medicament for oral administration is selected from the group consisting of tablets, capsules, cachets, powders, granules, or suspensions in a liquid.

27. The use according to claim 25 or claim 26, wherein the oxa compound of formula I is in an amount of from 1 to 100 mg per medicament unit.

28. The use according to claim 27, wherein the medicament is a tablet, where the oxalate salt of the compound of formula I is in an amount of from 1 to 100 mg per tablet unit.

29. The use according to any one of claims 21-24, wherein the medicament is for topical administration.

30. The use according to claim 29, wherein the medicament for topical administration is selected from the group consisting of creams, oils, ointments, emulsions, gels, aqueous solutions, spray solutions and plasters.

31. The use according to claim 29 or claim 30, wherein the oxalate salt of the compound of formula I is in an amount of from 1 to 100 mg per medicament unit.

32. The use according to any one of claims 21-24, wherein the medicament is for parenteral administration.

33. The use of an acid addition salt according to claim 32, wherein the medicament for parenteral administration is a sterile aqueous preparation.

34. The use of an acid addition salt according to claim 32 or claim 33, wherein the oxalate salt of the compound of formula I is in an amount of from 0.1 to 100 mg per medicament unit.

35. Oxalate salt of the compound of formula I 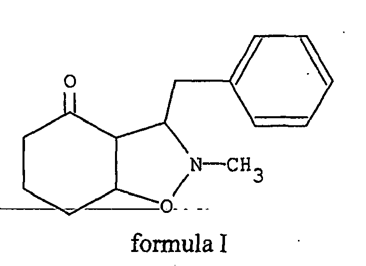 for use in the treatment of mood disorders, disorders of anxiety, depression and convulsive conditions, in the improvement of learning ability, in the reversal of amnesia, in resolving the abstinence syndrome from drugs and drugs of abuse.

## Patentansprüche

1. Oxalatsalz einer Verbindung gemäß der Formel I:

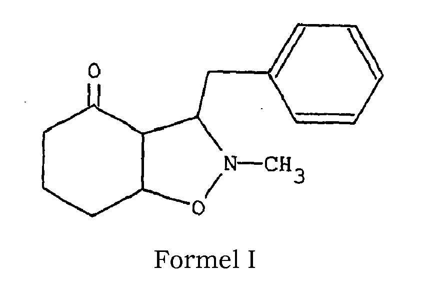

2. Oxalatsalz nach Anspruch 1, welches das Oxalatsalz einer Verbindung gemäß der Formel I in der Form einer oder mehrerer stereochemischer Verbindungen und Mischungen hiervon ist.

3. Oxalatsalz nach Anspruch 2, welches das Oxalatsalz einer Verbindung gemäß der Formel I in der Form eines Diastereoisomers oder einer racemischen Mischung ist.

4. Oxalatsalz nach einem der Ansprüche 1 bis 3, welches Oxalat von rel-(3R,3aS,7aS)-3-Benzyl-2-methyl-2,3,3a,4,5,6,7,7a-octahydrobenzo[d]isoxazol-4-on ist.

5. Verfahren zum Herstellen eines Oxalatsalzes von 3-Benzyl-2-methyl-2,3,3a,4,5,6,7,7a-octahydrobenzo[d]isoxazol-4-on nach einem der Ansprüche 1 bis 4, welches die nachfolgenden Schritte umfasst:
i) Reagieren der freien Base 3-Benzyl-2-methyl-2,3,3a,4,5,6,7,7a-octahydrobenzo[d]isoxazol-4-on mit Oxalsäure,
ii) Unterwerfen der Reaktionsmischung gegenüber einem oder mehreren Abkühlzyklen bei weniger als 10°C.

6. Verfahren nach Anspruch 5, wobei die freie Base 3-Benzyl-2-methyl-2,3,3a,4,5,6,7,7a-octahydrobenzo[d]isoxazol-4-on gemäß dem Schritt i) in einem Schritt vor dem Schritt i) erhalten wird, bei welchem die Base aus dem entsprechenden Hydrochloridsalz freigesetzt wird.

7. Verfahren nach Anspruch 6, wobei das Freisetzen der freien Base aus dem Hydrochloridsalz mittels Dichlormethan durchgeführt wird.

8. Verfahren nach einem der Ansprüche 5 bis 7, wobei die Oxalsäure des Schritts i) in der Dihydratform vorliegt.

9. Verfahren nach einem der Ansprüche 5 bis 8, wobei der Schritt i) in einem Eisbad durchgeführt wird.

10. Verfahren nach einem der Ansprüche 5 bis 9, wobei das Abkühlen gemäß dem Schritt ii) bei Temperaturen zwischen 2 und 8°C durchgeführt wird.

11. Oxalatsalz einer Verbindung gemäß der Formel 1 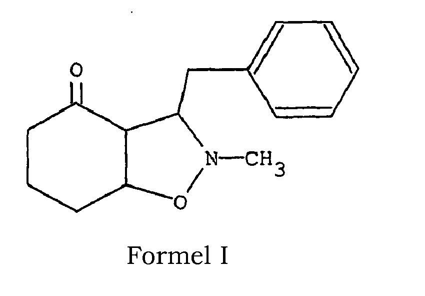 zur Verwendung als Arzneimittel.

12. Oxalatsalz nach Anspruch 11, welches das Oxalatsalz einer Verbindung gemäß der Formel I in der Form einer oder mehrerer stereochemischer Verbindungen und Mischungen hiervon zur Verwendung als ein Arzneimittel ist.

13. Oxalatsalz nach Anspruch 12, welches das Oxalatsalz einer Verbindung gemäß der Formel I in der Form eines Diastereoisomers oder einer racemischen Mischung zur Verwendung als ein Arzneimittel ist.

14. Oxalatsalz nach einem der Ansprüche 11 bis 13, welches das Oxalat von rel-(3R,3aS,7aS)-3-Benzyl-2-methyl-2,3,3a,4,5,6,7,7a-octahydrobenzo[d]isoxazol-4-on zur Verwendung als ein Arzneimittel ist.

15. Pharmazeutische Zusammensetzung enthaltend ein Oxalatsalz nach einem der Ansprüche 1 bis 4 sowie einen pharmazeutisch akzeptablen Träger.

16. Zusammensetzung nach Anspruch 15, wobei das Oxalatsalz in einer Menge zwischen 0,1 und 100 mg vorliegt.

17. Tablette, welche ein Oxalatsalz nach einem der Ansprüche 1 bis 4 und geeignete Hilfsstoffe enthält.

18. Tablette nach Anspruch 17, wobei die geeigneten Hilfsstoffe aus der Gruppe ausgewählt sind, welche aus Pregel-Stärke, mikrokristalliner Cellulose, Natriumstärkeglykolat, Talk, Lactose, Magnesiumstearat, Saccharose, Stearinsäure und Mannit besteht.

19. Tablette nach Anspruch 17 oder Anspruch 18, wobei das Oxalatsalz in einer Menge zwischen 1 und 100 mg pro Tabletteneinheit vorliegt.

20. Tablette nach Anspruch 19, wobei das Oxalatsalz in einer Menge zwischen 1 und 50 mg pro Tabletteneinheit vorliegt.

21. Verwendung eines Oxalatsalzes einer Verbindung gemäß der Formel I 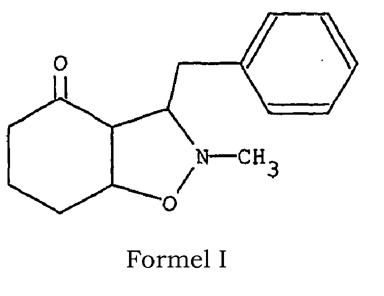 zur Herstellung eines Arzneimittels zur Behandlung von Gemütserkrankungen, Angststörungen, Depression und konvulsiver Zustände, bei der Verbesserung der Lernfähigkeit, bei der Umkehrung von Amnesie, bei dem Lösen des Abstinenzsyndroms von Arzneimitteln und Drogenmissbrauch.

22. Verwendung nach Anspruch 21, wobei das Oxalatsalz Oxalat einer Verbindung gemäß der Formel I in der Form einer oder mehrerer stereochemischer Verbindungen und Mischungen hiervon für die Herstellung eines Arzneimittels zur Behandlung von Gemütserkrankungen, Angststörungen, Depression und konvulsiver Zustände, bei der Verbesserung der Lernfähigkeit, bei der Umkehrung von Amnesie, bei dem Lösen des Abstinenzsyndroms von Arzneimitteln und Drogenmissbrauch ist.

23. Verwendung nach Anspruch 22, wobei das Oxalatsalz Oxalat einer Verbindung gemäß der Formel I in der Form eines Diastereoisomers oder einer racemischen Mischung für die Herstellung eines Arzneimittels zur Behandlung von Gemütserkrankungen, Angststörungen, Depression und konvulsiver Zustände, bei der Verbesserung der Lernfähigkeit, bei der Umkehrung von Amnesie, bei dem Lösen des Abstinenzsyndroms von Arzneimitteln und Drogenmissbrauch ist.

24. Verwendung nach einem der Ansprüche 21 bis 23, wobei das Oxalatsalz Oxalat von rel-(3R,3aS,7aS)-3-Benzyl-2-methyl-2,3,3a,4,5,6,7,7a-octahydrobenzo[d]isoxazol-4-on für die Herstellung eines Arzneimittels zur Behandlung von Gemütserkrankungen, Angststörungen, Depression und konvulsiver Zustände, bei der Verbesserung der Lernfähigkeit, bei der Umkehrung von Amnesie, bei dem Lösen des Abstinenzsyndroms von Arzneimitteln und Drogenmissbrauch.

25. Verwendung nach einem der Ansprüche 21 bis 24, wobei das Arzneimittel zur oralen Verabreichung ist.

26. Verwendung nach Anspruch 25, wobei das Arzneimittel für die orale Verabreichung aus der Gruppe ausgewählt ist, welche aus Tabletten, Kapseln, Kapseln aus Stärkemasse, Pulver, Granulaten oder Suspensionen in einer Flüssigkeit besteht.

27. Verwendung nach Anspruch 25 oder Anspruch 26, wobei die Oxaverbindung gemäß der Formel I in einer Menge zwischen 1 und 100 mg pro Arzneimitteleinheit vorliegt.

28. Verwendung nach Anspruch 27, wobei das Arzneimittel eine Tablette ist, wobei das Oxalatsalz der Verbindung gemäß der Formel I in einer Menge von 1 bis 100 mg pro Tabletteneinheit vorliegt.

29. Verwendung nach einem der Ansprüche 21 bis 24, wobei das Arzneimittel für die topische Verabreichung ist.

30. Verwendung nach Anspruch 29, wobei das Arzneimittel für die topische Verabreichung aus der Gruppe ausgewählt wird, welche aus Cremes, Ölen, Salben, Emulsionen, Gelen, wässrigen Lösungen, Sprühlösungen und Pflastern besteht.

31. Verwendung nach Anspruch 29 oder Anspruch 30, wobei das Oxalatsalz der Verbindung gemäß der Formel I in einer Menge von 1 bis 100 mg pro Arzneimitteleinheit vorliegt.

32. Verwendung nach einem der Ansprüche 21 bis 24, wobei das Arzneimittel für die parenterale Verabreichung ist.

33. Verwendung eines Säurezugabesalzes nach Anspruch 32, wobei das Arzneimittel für die parenterale Verabreichung eine sterile wässrige Zubereitung ist.

34. Verwendung eines Säurezugabesalzes nach Anspruch 32 oder Anspruch 33, wobei das Oxalatsalz der Verbindung gemäß der Formel I in einer Menge von 0,1 bis 100 mg pro Arzneimitteleinheit vorliegt.

35. Oxalatsalz einer Verbindung gemäß der Formel 1 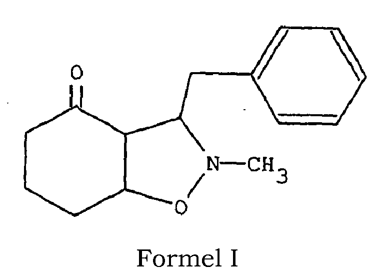 zur Verwendung bei der Behandlung von Gemütserkrankungen, Angststörungen, Depression und konvulsiver Zustände, bei der Verbesserung der Lernfähigkeit, bei der Umkehrung von Amnesie, bei dem Lösen des Abstinenzsyndroms von Arzneimitteln und Drogenmissbrauch.

## Revendications

1. Sel d'oxalate du composé de formule I

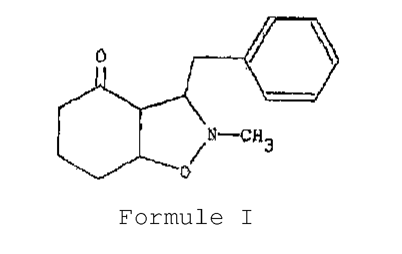

2. Sel d'oxalate selon la revendication 1 qui est un sel d'oxalate du composé de formule I sous la forme d'un ou plusieurs composés stéréochimiques et leurs mélanges.

3. Sel d'oxalate selon la revendication 2 qui est un sel d'oxalate du composé de formule I sous la forme d'un diastéréoisomère ou d'un mélange racémique.

4. Sel d'oxalate selon l'une quelconque des revendications 1 à 3, qui est l'oxalate de rel-(3R,3aS,7aS)-3-benzyl-2-méthyl-2,3,3a,4,5,6,7,7a-octahydrobenzo[d]isoxazol-4-one.

5. Procédé de préparation de sel d'oxalate de 3-benzyl-2-méthyl-2,3,3a,4,5,6,7,7a-octahydrobenzo[d] isoxazol-4-one, selon l'une quelconque des revendications 1 à 4, comprenant les étapes suivantes consistant à :
i) faire réagir la base libre 3-benzyl-2-méthyl-2,3,3a,4,5,6,7,7a-octahydrobenzo[d]isoxazol-4-one avec de l'acide oxalique ;
ii) soumettre le mélange de réaction à un ou plusieurs cycles de refroidissement inférieurs à 10 °C.

6. Procédé selon la revendication 5, dans lequel la base libre 3-benzyl-2-méthyl-2,3,3a,4,5,6,7,7a-octahydrobenzo[d]isoxazol-4-one de l'étape i) est obtenue dans une étape préalable à l'étape i), qui assure l'élimination dans ladite base du sel de chlorhydrate correspondant.

7. Procédé selon la revendication 6, dans lequel l'élimination du sel de chlorhydrate dans la base libre est réalisée par du dichlorométhane.

8. Procédé selon l'une quelconque des revendications 5 à 7, dans lequel l'acide oxalique de l'étape i) est sous forme de dihydrate.

9. Procédé selon l'une quelconque des revendications 5 à 8, dans lequel l'étape i) est réalisée dans un bain de glace.

10. Procédé selon l'une quelconque des revendications 5 à 9, dans lequel le refroidissement de l'étape ii) s'effectue à des températures de 2 à 8 °C.

11. Sel d'oxalate du composé de formule I 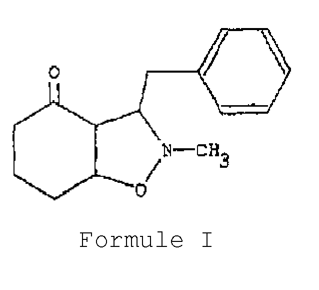 à utiliser comme médicament.

12. Sel d'oxalate selon la revendication 11, qui est un sel d'oxalate du composé de formule I sous la forme d'un ou plusieurs composés stéréochimiques et leurs mélanges à utiliser comme médicament.

13. Sel d'oxalate selon la revendication 12, qui est un sel d'oxalate du composé de formule I sous la forme d'un diastéréoisomère ou d'un mélange racémique à utiliser comme médicament.

14. Sel d'oxalate selon l'une quelconque des revendications 11 à 13, qui est l'oxalate de rel-(3R,3aS,7aS)-3-benzyl-2-méthyl-2,3,3a,4,5,6,7,7a-octahydrobenzo [d] isoxazol-4-one à utiliser comme médicament.

15. Composition pharmaceutique comprenant le sel d'oxalate selon l'une quelconque des revendications 1 à 4 et un support pharmaceutiquement acceptable.

16. Composition selon la revendication 15, dans laquelle le sel d'oxalate est présent dans une quantité de 0,1 à 100 mg.

17. Comprimé comprenant le sel d'oxalate selon l'une quelconque des revendications 1 à 4 et des excipients appropriés.

18. Comprimé selon la revendication 17, dans lequel les excipients appropriés sont choisis dans le groupe constitué par l'amidon prégélatinisé, la cellulose microcristalline, le glycolate d'amidon sodique, le talc, le lactose, le stéarate de magnésium, le saccharose, l'acide stéarique et le mannitol.

19. Comprimé selon la revendication 17 ou la revendication 18, dans lequel le sel d'oxalate est présent dans une quantité de 1 à 100 mg par comprimé unitaire.

20. Comprimé selon la revendication 19, dans lequel le sel d'oxalate est présent dans une quantité de 1 à 50 mg par comprimé unitaire.

21. Utilisation du sel d'oxalate du composé de formule I 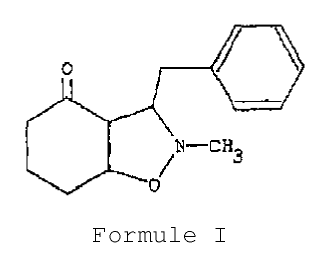 pour la fabrication d'un médicament destiné à traiter les troubles de l'humeur, les troubles de l'anxiété, la dépression et les affections convulsives, à améliorer l'aptitude à l'apprentissage, à faire régresser l'amnésie, à résoudre le syndrome de sevrage des médicaments et des drogues.

22. Utilisation selon la revendication 21, dans laquelle le sel d'oxalate est l'oxalate du composé de formule I sous la forme d'un ou plusieurs composés stéréochimiques et leurs mélanges pour la fabrication d'un médicament destiné à traiter les troubles de l'humeur, les troubles de l'anxiété, la dépression et les affections convulsives, à améliorer l'aptitude à l'apprentissage, à faire régresser l'amnésie, à résoudre le syndrome de sevrage des médicaments et des drogues.

23. Utilisation selon la revendication 22, dans laquelle le sel d'oxalate est l'oxalate du composé de formule I sous la forme d'un diastéréoisomère ou d'un mélange racémique pour la fabrication d'un médicament destiné à traiter les troubles de l'humeur, les troubles de l'anxiété, la dépression et les affections convulsives, à améliorer l'aptitude à l'apprentissage, à faire régresser l'amnésie, à résoudre le syndrome de sevrage des médicaments et des drogues.

24. Utilisation selon l'une quelconque des revendications 21 à 23, dans laquelle le sel d'oxalate est l'oxalate de rel-(3R,3aS,7aS)-3-benzyl-2-méthyl-2, 3, 3a, 4, 5, 6, 7, 7a-octahydrobenzo [d] isoxazol-4-one pour la fabrication d'un médicament destiné à traiter les troubles de l'humeur, les troubles de l'anxiété, la dépression et les affections convulsives, à améliorer l'aptitude à l'apprentissage, à faire régresser l'amnésie, à résoudre le syndrome de sevrage des médicaments et des drogues.

25. Utilisation selon l'une quelconque des revendications 21 à 24, dans laquelle le médicament est destiné à une administration par voie orale.

26. Utilisation selon la revendication 25, dans laquelle le médicament pour une administration par voie orale est choisi dans le groupe constitué par les comprimés, les capsules, les cachets, les poudres, les granules ou les suspensions dans un liquide.

27. Utilisation selon la revendication 25 ou la revendication 26, dans laquelle le composé oxa de formule I se trouve dans une quantité de 1 à 100 mg par médicament unitaire.

28. Utilisation selon la revendication 27, dans laquelle le médicament est un comprimé, où le sel d'oxalate du composé de formule I se trouve dans une quantité de 1 à 100 mg par comprimé unitaire.

29. Utilisation selon l'une quelconque des revendications 21 à 24, dans laquelle le médicament est destiné à une administration par voie topique.

30. Utilisation selon la revendication 29, dans laquelle le médicament destiné à une administration par voie topique est choisi dans le groupe constitué par les crèmes, les huiles, les onguents, les émulsions, les gels, les solutions aqueuses, les solutions de pulvérisation et les emplâtres.

31. Utilisation selon la revendication 29 ou la revendication 30, dans laquelle le sel d'oxalate du composé de formule I se trouve dans une quantité de 1 à 100 mg par médicament unitaire.

32. Utilisation selon l'une quelconque des revendications 21 à 24, dans laquelle le médicament est destiné à une administration par voie parentérale.

33. Utilisation d'un sel d'addition d'acide selon la revendication 32, dans laquelle le médicament destiné à une administration par voie parentérale est une préparation aqueuse stérile.

34. Utilisation d'un sel d'addition d'acide selon la revendication 32 ou la revendication 33, dans laquelle le sel d'oxalate du composé de formule I se trouve dans une quantité de 0,1 à 100 mg par médicament unitaire.

35. Sel d'oxalate du composé de formule I 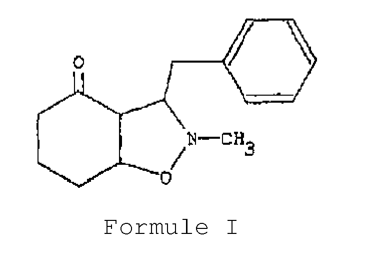 à utiliser pour traiter les troubles de l'humeur, les troubles de l'anxiété, la dépression et les affections convulsives, pour améliorer l'aptitude à l'apprentissage, pour faire régresser l'amnésie, pour résoudre le syndrome de sevrage des médicaments et des drogues.
